# EUROPÄISCHE PATENTSCHRIFT

(11) **EP 1 616 858 B1**
(45) Veröffentlichungstag und Bekanntmachung des Hinweises auf die Patenterteilung: **21.05.2008**
(21) Anmeldenummer: 05014272.8
(22) Anmeldetag: 30.06.2005
(51) Int. Cl.: C07C 267/00, C08G 18/02

(54) **Verfahren zur Herstellung flüssiger, lagerstabiler Carbodiimid- und/oder Uretonimingruppen aufweisender organischer Isocyanate mit niedriger Farbzahl**
Process for the preparation of fluid and storage stable organic isocyanates containing carbodiimide and/or uretonimino groups with low colour index
Procédé pour la préparation d'isocyanates organiques liquides, stables au stockage, contenant des groupes carbodiimido et/ou uretonimino ayant un indice de couleur faible

(30) Priorität: 13.07.2004 DE 102004033849
(43) Veröffentlichungstag der Anmeldung: 18.01.2006
(73) Patentinhaber: Bayer MaterialScience AG, 51368 Leverkusen (DE)
(72) Erfinder: Wershofen, Stefan, Dr., 41065 Mönchengladbach (DE); Schmidt, Manfred, Dr., 41540 Dormagen (DE); Pirkl, Hans-Georg, Dr., 51377 Leverkusen (DE); Kamiyama, Nobuhiro, Itami, Hyogo (JP); Murakami, Shinichi, Sanda, Hyogo (JP); Harada, Tetsuo, Kobe, Hyogo (JP)

(56) Entgegenhaltungen:
- EP-A- 0 515 933
- US-A- 4 088 665
- US-A- 6 120 699

## Beschreibung

Die Erfindung betrifft ein Verfahren zur Herstellung flüssiger, lagerstabiler Carbodiimid- (CD) und/oder Uretonimin- (UI) Gruppen aufweisender Isocyanatmischungen mit niedriger Farbzahl, die nach diesem Verfahren erhältlichen Isocyanatmischungen und deren Verwendung zur Herstellung von Abmischungen mit weiteren Isocyanaten bzw. zur Herstellung von Isocyanatgruppen enthaltenden Prepolymeren sowie von Polyurethankunststoffen, vorzugsweise Polyurethanschaumstoffen.

CD- und /oder UI-Gruppen aufweisende Isocyanatmischungen können in einfacher Weise mit den hoch wirksamen Katalysatoren aus der Phospholin-Reihe, insbesondere der Phospholinoxid-Reihe, nach den Verfahren gemäß US-A-2,853,473 und EP-A-515 933 US-A-6,120,699 hergestellt werden.

Die hohe katalytische Aktivität der Phospholinkatalysatoren, insbesondere der Phospholinoxidkatalysatoren, ist einerseits erwünscht, um die Carbodiimidisierungsreaktion unter schonenden Temperaturbedingungen anzustoßen, andererseits ist aber bis heute kein Verfahren bekannt, das eine wirksame Abstoppung der Phospholin-Katalyse bzw. der Phospholinoxid-Katalyse ohne Einschränkung gewährleistet. Die carbodiimidisierten Isocyanate neigen zur Nachreaktion, d.h. sie gasen infolge CO₂-Entwicklung aus. Dies führt dann besonders bei einem Temperaturanstieg zu einem Druckaufbau beispielsweise in den Lagerbehältern.

Es hat nicht an Versuchen gefehlt, eine wirksame Abstoppung der Phospholin-Katalyse zu finden. Geeignete Stopper sind z.B. in den Patentschriften EP-A-515 933, EP-A-609 698 und US-A-6,120,699 erwähnt und umfassen z.B. Säuren, Säurechloride, Chloroformiate und silylierte Säuren. Ein Abstoppen des Katalysators mit Säuren, die z.B. auch als Säurechloride vorliegen können, ist nicht ausreichend wirksam.

Nach der Lehre der EP-A-515 933 werden mittels Phospholin-Katalyse hergestellte CD/UI-haltige Isocyanatmischungen mit mindestens der äquimolaren Menge, bevorzugt der 1-2fachen molaren Menge bezogen auf den eingesetzten Katalysator an z.B. Trimethylsilyltrifluormethansulfonat (TMST) abgestoppt. In der Praxis hat sich jedoch erwiesen, dass solchermaßen hergestellte CD/UI-enthaltende Isocyanate zur Herstellung von Prepoymeren, d.h. Umsetzungsprodukten von diesen CD/UI-enthaltenden Isocyanaten mit Polyolen, nur bedingt geeignet sind. Die entsprechend hergestellten Umsetzungsprodukte aus Polyolen und den CD/UI-modifizierten Isocyanaten neigen zum Ausgasen, was zu einem Druckaufbau in den Transportbehältern oder zum Schäumen beim Handling derartiger Produkte führen kann.

Man kann dieses Problem dadurch umgehen, dass man die zum Abstoppen des Phospholin-Katalysators benutzte silylierte Säure analog der EP-A-515 933 in höheren molaren Äquivalenten (z.B. 5:1-10:1 bezogen auf den Katalysator) einsetzt. In der Praxis zeigt sich dann jedoch, dass die erhaltenen CD/UI modifizierten Isocyanate eine deutlich schlechtere Farbzahl aufweisen. Dies gilt dann auch für die hieraus hergestellten Prepolymere.

Dies gilt auch, wenn der Phospholinkatalysator mit Säuren vom Typ der Trifluormethansulfonsäure entsprechend der US-A-6,120,699 abgestoppt werden. Auch daraus hergestellte Prepolymere weisen eine erheblich erhöhte Farbzahl auf.

Aufgabe der vorliegenden Erfindung war es daher, ein Verfahren zur Herstellung flüssiger, lagerstabiler Carbodiimid- und/oder Uretonimingruppen aufweisender Isocyanatmischungen zur Verfügung zu stellen, welches die angesprochenen Mängel nicht aufweist und zu flüssigen, lagerstabilen Isocyanatmischungen mit niedrigen Farbzahlen führt.

Die Erfindung betrifft ein Verfahren zur Herstellung Carbodiimid- und/oder Uretonimingruppen aufweisender organischer Isocyanate durch teilweise Carbodiimidisierung von Isocyanatgruppen mit Katalysatoren vom Phospholin-Typ, und anschließendes Abstoppen der Carbodiimidisierungsreaktion durch den Zusatz einer silylierten Säure der Formel X-[Si(CH₃)₃]ₙ, wobei in der Formel
- X: für den neutralen Säurerest steht, wie er durch Entfernen der aziden Wasserstoffatome aus einer n- basischen Säure mit einem pKa-Wert von max. 3 erhalten wird, wobei Halogenwasserstoffsäuren ausgenommen sind, und
- n: eine ganze Zahl von 1 - 3 bedeutet,
dadurch gekennzeichnet, dass zusätzlich zu der silylierten Säure eine nicht silylierte Säure und/oder ein Säurechlorid und/oder ein Sulfonsäureester zugesetzt wird, wobei als nicht silylierte Säure gegebenenfalls halogenierte, aliphatische und/oder cycloaliphatische und/oder aromatische Mono-, Di- und/oder Poly-Carbonsäuren, Sulfonsäuren, HCl und/oder Phosphorsäure bzw. deren Mono-und/oder Diester, eingesetzt werden und als Säurechlorid die aus den gegebenenfalls halogenierten, aliphatischen und/oder cycloaliphatischen und/oder aromatischen Mono-, Di- und/oder Poly-Carbonsäuren bzw. Sulfonsäuren abgeleiteten Säurechloride sowie Carbamidsäurechloride eingesetzt werden.

Bevorzugt werden als silylierte Säuren der Formel X-[Si(CH₃)₃]ₙ O-silylierte, Sauerstoff enthaltende Säuren verwendet, die in nicht silylierter Form einen pKa-Wert von maximal 3 aufweisen wie beispielsweise Trifluormethansulfonsäuretrimethylsilylester (Trimethylsilyltrifluormethansulfonat, TMST).

Die Erfindung betrifft auch die Carbodiimid- und/oder Uretonimingruppen aufweisenden organischen Isocyanate, die nach dem obengenannten Verfahren erhältlich sind. Diese Carbodiimid- und/oder Uretonimingruppen aufweisenden organische Isocyanate sind bei Raumtemperatur und in Abhängigkeit vom CD- / UI-Gehalt und/oder vom eingesetzten Isocyanat bis hin zu tiefen Temperaturen (z.B. 0°C) flüssig.

Gegenstand der Erfindung ist auch die Verwendung der erfindungsgemäßen Carbodiimid- und/oder Uretonimingruppen aufweisenden organischen Isocyanate zur Herstellung von Abmischungen mit weiteren Isocyanaten bzw. zur Herstellung von Isocyanatgruppen enthaltenden Prepolymeren mit verbesserter Farbzahl.

Gegenstand der Erfindung ist schließlich auch die Verwendung der erfindungsgemäßen Carbodiimid- und/oder Uretonimingruppen aufweisenden organischen Isocyanate und der daraus hergestellten Isocyanat-Abmischungen und/oder Prepolymere mit verbesserter Farbzahl zur Herstellung von Polyurethankunststoffen.

Als Ausgangsmaterialien für das erfindungsgemäße Verfahren können beliebige organische Isocyanate eingesetzt werden. Vorzugsweise wird das erfindungsgemäße Verfahren jedoch zur Carbodiimidisierung von organischen Diisocyanaten verwendet, die in der Polyurethan-Chemie eingesetzt werden.

Geeignet sind insbesondere
- Aromatische Diisocyanate wie 2,4- und/oder 2,6-Diisocyanatotoluol (TDI), 2,2'- ,2,4'- und/oder 4,4'-Diisocyanatodiphenylmethan (MDI) bzw. beliebige Gemische derartiger aromatischer Diisocyanate,
- Polyisocyanatgemische der Diphenylmethanreihe mit einem Gehalt an monomeren Diisocyanatodiphenylmethan-Isomeren von 80 bis 100 Gew.-% und 0 bis 20 Gew.-% an höher als difunktionellen Polyisocyanaten der Diphenylmethanreihe, wobei sich die Diisocyanatodiphenylmethan-Isomeren zu 0 bis100 Gew.-% aus 4,4'-Diisocyanatodiphenylmethan, 100 bis 0 Gew,-% aus 2,4'-Diisocyanatodiphenylmethan und zu 0-8 Gew.-% aus 2,2'-Diisocyanatodiphenylmethan zusammensetzen können, wobei sich die genannten Prozentsätze zu 100% ergänzen.
- Polyphenylpolymethylenpolyisocyanate, die durch Anilin-Formaldehyd-Kondensation und anschließende Phosgenierung hergestellt werden ("rohes MDI").

Das erfindungsgemäße Verfahren wird in Gegenwart von Katalysatoren vom Phospholin-Typ durchgeführt. Die Katalysatoren vom Phospholin-Typ sind beispielsweise aus EP-A-515 933 und US-A-6,120,699 bekannt. Typische Beispiele dieser Katalysatoren sind beispielsweise die aus dem Stand der Technik bekannten Gemische der Phospholinoxide der Formel:

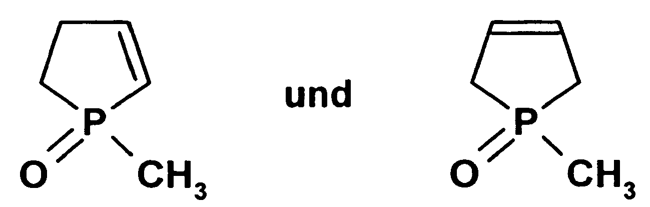

Die Menge des eingesetzten Katalysators hängt von der Qualität der Ausgangsisocyanate ab. Die jeweils notwendige Katalysatorenmenge lässt sich daher am einfachsten in einem Vorversuch bestimmen.

Die Carbodiimidisierungsreaktion wird üblicherweise im Temperaturbereich zwischen 50 bis 150°C, vorzugsweise von 60 bis 100°C, durchgeführt. Die optimale Reaktionstemperatur richtet sich nach der Art der Ausgangsisocyanate und kann in einem einfachen Vorversuch ermittelt werden.

Die Carbodiimidisierungsreaktion wird im allgemeinen bei Erreichen eines Carbodiimidisierungsgrades (Carbodiimidisierungsgrad ist der Prozentsatz der carbodiimidisierten Isocyanatgruppen bezogen auf die Gesamtmenge der in Ausgangsisocyanat vorliegenden Isocyanatgruppen) von 3 bis 50%, vorzugsweise 5 bis 30%, abgebrochen.

Der Carbodiimidisierungsgrad kann während der Durchführung des erfindungsgemäßen Verfahrens durch Bestimmung des NCO-Wertes z.B. mittels dem Fachmann an sich bekannter Titration oder mittels online-Verfahren bestimmt werden. Ein geeignetes online-Verfahren ist z.B. die Nahinfrarot- oder die Mittelinfrarot-Analytik.

Der Carbodiimidisierungsgrad kann während der Durchführung des erfindungsgemäßen Verfahrens ebenfalls z.B. an der Menge des im Reaktorgemisch entweichenden Kohlendioxids erkannt werden. Diese volumetrisch bestimmbare Kohlendioxidmenge gibt somit zu jedem Zeitpunkt Auskunft über den erreichten Carbodiimidisierungsgrad.

Darüber hinaus können grundsätzlich auch andere geeignete, dem Fachmann bekannte offline- oder online-Methoden der Prozessverfolgung eingesetzt werden.

Zum Beenden der Carbodiimidisierungsreaktion wird als Abstopper bevorzugt mindestens die äquimolaren Menge, besonders bevorzugt der 1 - 5 fache molare Überschuss, ganz besonders bevorzugt der 1 - 3 fache molare Überschuss, bezogen auf den Katalysator, einer silylierten Säure der Formel X-[Si(CH₃)₃]ₙ eingesetzt, wobei X und n die bereits oben genannte Bedeutung haben.

Geeignet sind beispielsweise silylierte Sulfonsäuren wie Trifluormethansulfonsäuretrimethylsilylester (TMST) oder Methansulfonsäuretrimethylsilylester, silylierte Ester von Säuren des Phosphors wie Phosphorsäuretris(trimethylsilylester) oder Phosphorsäurediethylester-trimethylsilylester.

Zusätzlich zu den silylierten Säuren als Abstopper wird in dem erfindungsgemäßen Verfahren als Stabilisator eine nicht silylierte Säure und/oder ein Säurederivat einer nicht silylierten Säure, insbesondere ein Säurechlorid und/oder ein Sulfonsäureester, zugesetzt. Die Zugabe dieses Stabilisators kann entweder zeitgleich mit der Zugabe des Abstoppers oder in einem nachgeschalteten Schritt erfolgen.

Als nicht silylierte Säuren können gegebenenfalls halogenierte, aliphatische und/oder cycloaliphatische und/oder aromatische Mono-, Di- und/oder Poly-Carbonsäuren wie z.B. Essigsäure, Adipinsäure, Cyclohexandicarbonsäure, α-Chlorpropionsäure, Benzoesäure, Phthalsäure, Isophtalsäure usw., sowie Sulfonsäuren, HCl und/oder Phosphorsäuren bzw. deren Mono-und/oder Diester wie z.B. Dibutylphosphat, eingesetzt werden. Als Säurechloride können die aus den gegebenenfalls halogenierten, aliphatischen und/oder cycloaliphatischen und/oder aromatischen Mono-, Di- und/oder Poly-Carbonsäuren bzw. Sulfonsäuren abgeleiteten Säurechloride sowie Carbamidsäurechloride wie z.B. n-Butylcarbamidsäurechlorid eingesetzt werden. Als Sulfonsäureester können z.B. p-Toluolsulfonsäuremethylester, p-Toluolsulfonsäureethylester eingesetzt werden.

Die zusätzlichen Stabilisatoren werden dabei in Mengen von insgesamt zwischen 10 und 1000 ppm, bevorzugt zwischen 10 und 500 ppm, besonders bevorzugt zwischen 50 und 250 ppm, bezogen auf die Carbodiimid- und/oder Uretonimingruppen aufweisenden organischen Isocyanate, zugesetzt.

Dabei hat sich gezeigt, dass durch die kombinierte Zugabe der silylierten Säure mit einem zusätzlichen Stabilisator enthaltend mindestens eine nicht silylierte Säure und/oder mindestens ein Säurechlorid und/oder mindestens einen Sulfonsäureester nach dem erfindungsgemäßen Verfahren sowohl die Phospholin-Katalyse wirksam abgestoppt werden kann, als auch gleichzeitig niedrige Farbwerte im erzeugten Isocyanat und daraus hergestellten Prepolymeren erhalten werden können. Bei alleinigem Einsatz der silylierten Säure als Abstopper gemäß dem Stand der Technik lässt sich die Phospholin-Katalyse nur durch Zugabe großer Mengen an silylierter Säure wirksam abstoppen, was aber zu erhöhten Farbwerten der so erzeugten Isocyanatmischungen und daraus hergestellten Prepolymeren führt. Gleiches gilt für die alleinige Zugabe einer nicht silylierten Säure, eines Säurechlorids oder eines Sulfonsäureesters. Die kombinierte Zugabe der silylierten Säure mit mindestens einer nicht silylierten Säure und/oder mindestens einem Säurechlorid und/oder mindestens einem Sulfonsäureester erzeugt daher eine synergistische Wirkung.

Isocyanatgruppen enthaltende Prepolymere werden durch Umsetzung der nach dem erfindungsgemäßen Verfahren hergestellten Carbodiimid- und/oder Uretonimingruppen aufweisenden organischen Isocyanate mit in der Polyurethanchemie üblichen Polyolen erhalten. Geeignete Polyole sind sowohl einfache mehrwertige Alkohole des Molekulargewichtsbereiches 62 bis 599 g/mol, vorzugsweise 62 bis 300 g/mol, wie z.B. Ethylenglykol, Trimethylolpropan, Propandiol-1,2, Butandiol-1,2 oder Butandiol-2,3, Hexandiol, Octandiol, Dodecandiol und/oder Octadecandiol, insbesondere jedoch höhermolekulare Polyetherpolyole und/oder Polyesterpolyole der aus der Polyurethanchemie an sich bekannten Art mit Molekulargewichten von 600 bis 8000 g/mol, vorzugsweise 800 bis 4000 g/mol, die mindestens zwei, in der Regel 2 bis 8, vorzugsweise 2 bis 4 primäre und/oder sekundäre Hydroxylgruppen aufweisen. Beispiele derartiger Polyole sind in der US-PS 4 218543, Kolonne 7, Zeile 29 bis Kolonne 9, Zeile 32 beschrieben

Die Vorteile des erfindungsgemäßen Verfahrens sind augenscheinlich: Sowohl die Carbodiimid und/oder Uretonimingruppen haltigen Isocyanate als auch die daraus hergestellten Prepolymere weisen eine gute Lagerstabilität und eine helle Farbe auf.

Diese Carbodiimid- und/oder Uretonimingruppen aufweisenden organischen Isocyanate und die daraus hergestellten Prepolymere stellen wertvolle Ausgangsmaterialien zur Herstellung von Polyurethankunststoffen nach dem Isocyanat-Polyadditionsverfahren dar.

### Beispiele:

### Ausgangsprodukte:

- Desmodur 44M^{®}, Bayer AG (4,4'- Diphenylmethandiisocyanat, NCO-Gehalt: 33,6 Gew.%)
- Katalysator vom Phospholinoxid-Typ: technisches Gemisch aus 1-Methyl-1-oxo-1-phosphacyclopent-2-en und 1-Methyl-1-oxo-1-phosphacyclopent-3-en, 10 %ig in Toluol
- Stopper: Trimethylsilyltrifuormethansulfonat (TMST)
- Polyol: Multranol^{®} 3901, Bayer Corp. (Polyether aus Propylen- und Ethylenoxydeinheiten mit 80 - 90% primären OH-Gruppen, einer Funktionalität von 3, einer OH-Zahl von 28 mg KOH/g und einer Viskosität von ca. 1200 mPas bei 25°C)

Allgemeine Vorschrift zur Herstellung des Carbodiimid- und/oder Uretonimingruppen aufweisenden organischen Isocyanates:

10 kg technisches 4,4'-MDI (Desmodur 44M^{®}) werden unter N₂/Rühren auf 60°C erhitzt und mit 352 mg der Katalysatorlösung (3,5 ppm; 0,3 mmol) versetzt. Das Reaktionsgemisch wird unter N₂/Rühren für 240 min auf 97°C erhitzt. Danach wird die Carbodiimidisierung durch Zugabe von 101 mg TMST (10 ppm; 0,45 mmol) abgestoppt und 1 Stunde nachgerührt. Anschließend wird in den erfindungsgemäßen Beispielen 1 bis 6 ein Stabilisator (Isophthalsäuredichlorid [IPDC] in Beispiel 1; α-Chlorpropionsäure [α-CIPS] in Beispiel 2; p-Toluolsulfonsäuremethylester [p-TSME] in Beispiel 3; gasförmige HCl [HCl] in Form eines Masterbatches in 4,4'-MDI in Beispiel 4; Sebazinsäuredichlorid [SDC] in Beispiel 5; Dibutylphosphat [DBP] in Beispiel 6) in den in der Tabelle angegebenen Mengen zugegeben. In den Vergleichsbeispielen 1 bis 3 wird auf die Zugabe des zusätzlichen Stabilisators verzichtet, jedoch wird in Vergleichsbeispiel 2 die TMST-Menge auf 55 ppm erhöht, und in Vergleichsbeispiel 3 wird anstelle von TMST Trifluormethansulfonsäure (70 ppm) als Stopper eingesetzt. Die Ergebnisse sind in der nachfolgenden Tabelle zusammengefasst.

Allgemeine Vorschrift zur Herstellung eines Prepolymeren aus Carbodiimid- und/oder Uretonimingruppen aufweisendem organischen Isocyanat und einem Polyol:

Jeweils 500 g des nach vorstehender Vorschrift hergestellten Isocyanates werden bei 50°C unter N₂/Rühren mit 167 g Multranol^{®} 3901 versetzt und weitere 2 h bei 80°C unter N₂/Rühren gehalten. Die analytische Charakterisierung der Prepolymeren erfolgt am nächsten Tag. Zur Beurteilung der Stabilität der Prepolymere werden isotherme Druckversuche (12 h / 90°C) durchgeführt. Die Ergebnisse sind in der nachstehenden Tabelle zusammengefasst.

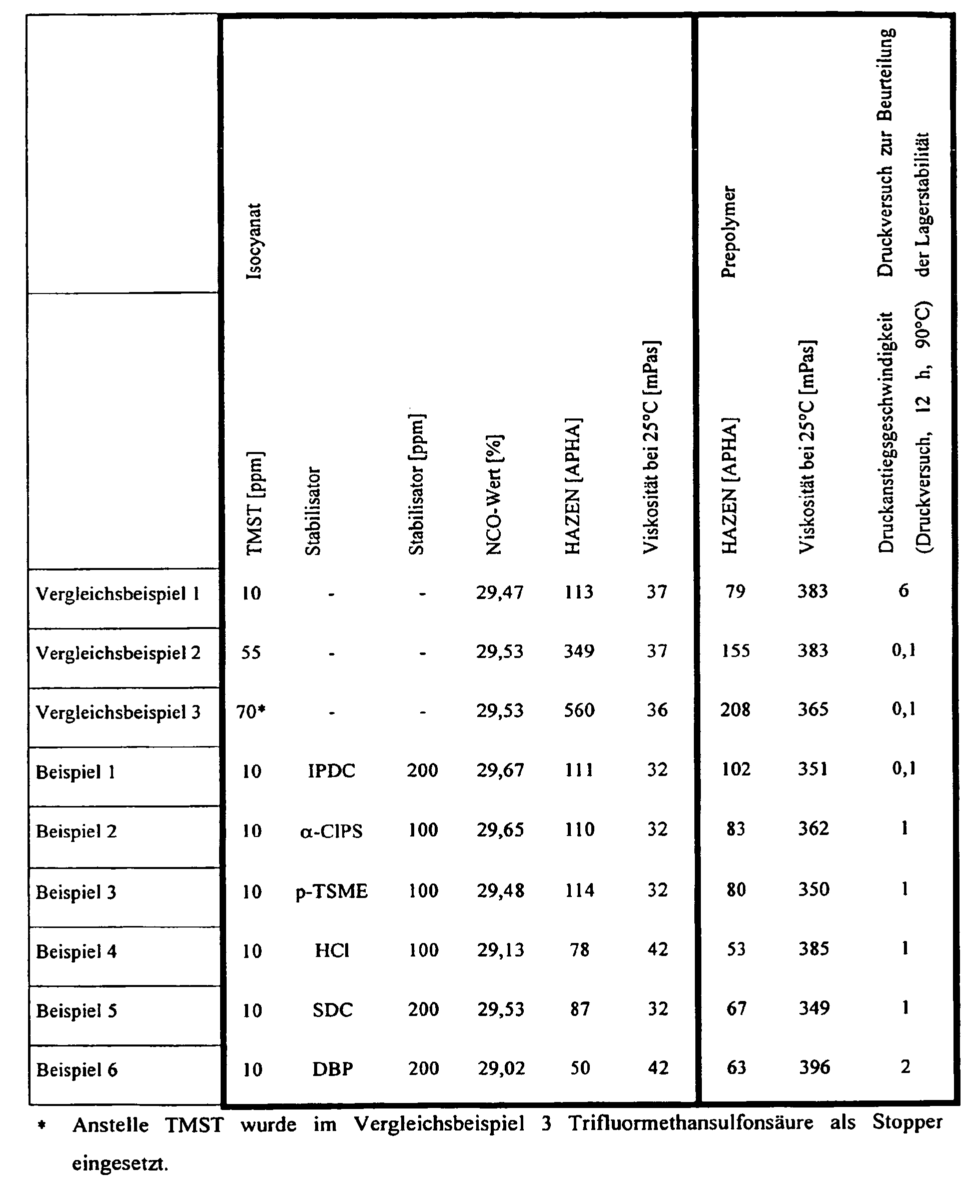

Vergleichsbeispiele 1 und 2 verdeutlichen den positiven Einfluss der erhöhten Stoppermenge an TMST auf die Stabilität, der jedoch zu Lasten der Farbe (HAZEN) geht. Vergleichsbeispiel 3 zeigt den nochmals ungünstigeren Einfluss des Stoppers Trifluormethansulfonsäure auf die Farbe (HAZEN). In den erfindungsgemäßen Beispielen 1 bis 6 wird eine gegenüber Vergleichsbeispiel 1 verbesserte Stabilität erreicht und dabei das gute Farb-Niveau (HAZEN) erhalten.

## Patentansprüche

1. Verfahren zur Herstellung Carbodiimid- und/oder Uretonimingruppen aufweisender organischer Isocyanate durch teilweise Carbodiimidisierung von Isocyanatgruppen mit Katalysatoren vom Phospholin-Typ, und anschließendes Abstoppen der Carbodiimidisierungsreaktion durch den Zusatz einer silylierten Säure der Formel X-[Si(CH₃)₃]ₙ, wobei in der Formel
X für den neutralen Säurerest steht, wie er durch Entfernen der aziden Wasserstoffatome aus einer n- basischen Säure mit einem pKa-Wert von max. 3 erhalten wird, wobei Halogenwasserstoffsäuren ausgenommen sind, und
n eine ganze Zahl von 1 - 3 bedeutet,
**dadurch gekennzeichnet, dass** zusätzlich zu der silylierten Säure eine nicht silylierte Säure und/oder ein Säurechlorid und/oder ein Sulfonsäureester zugesetzt wird, wobei als nicht silylierte Säure gegebenenfalls halogenierte, aliphatische und/oder cycloaliphatische und/oder aromatische Mono-, Di- und/oder Poly-Carbonsäuren, Sulfonsäuren, HCl und/oder Phosphorsäure bzw. deren Mono-und/oder Diester, eingesetzt werden und als Säurechlorid die aus den gegebenenfalls halogenierten, aliphatischen und/oder cycloaliphatischen und/oder aromatischen Mono-, Di- und/oder Poly-Carbonsäuren bzw. Sulfonsäuren abgeleiteten Säurechloride sowie Carbamidsäurechloride eingesetzt werden.

2. Verfahren nach Anspruch 1, **dadurch gekennzeichnet, dass** die Zugabe einer nicht silylierten Säure und/oder eines Säurechlorids und/oder eines Sulfonsäureesters entweder zeitgleich mit der Zugabe der silylierten Säure oder in einem nachgeschalteten Schritt erfolgen.

3. Verfahren nach einem der Ansprüche 1 oder 2, **dadurch gekennzeichnet, dass** die nicht silylierte Säure und/oder das Säurechlorid und/oder der Sulfonsäureester in Mengen von insgesamt zwischen 10 und 1000 ppm, bevorzugt zwischen 10 und 500, besonders bevorzugt zwischen 50 und 250 ppm, bezogen auf die Carbodiimid- und/oder Uretonimingruppen aufweisenden organischen Isocyanate, zugesetzt werden.

4. Verfahren nach Anspruch 1, **dadurch gekennzeichnet, dass** als nicht silylierte Säure Essigsäure, Adipinsäure, Cyclohexandicarbonsäure, α-Chlorpropionsäure, Benzoesäure, Phthalsäure, Isophtalsäure und/oder Dibutylphosphat zugesetzt werden.

5. Verfahren nach Anspruch 1, **dadurch gekennzeichnet, dass** als Säurechlorid n-Butylcarbamidsäurechlorid zugesetzt wird.

6. Verfahren nach einem der Ansprüche 1 bis 3, **dadurch gekennzeichnet, dass** als Sulfonsäureester p-Toluolsulfonsäuremethylester und/oder p-Toluolsulfonsäureethylester eingesetzt werden.

7. Carbodiimid- und/oder Uretonimingruppen aufweisende organische Isocyanate erhältlich nach dem Verfahren nach einem der Ansprüche 1 bis 6.

8. Verwendung der Carbodiimid- und/oder Uretonimingruppen aufweisenden organischen Isocyanate nach Anspruch 7 zur Herstellung von Isocyanat-Abmischungen.

9. Verwendung der Carbodiimid- und/oder Uretonimingruppen aufweisenden organischen Isocyanate nach Anspruch 7 zur Herstellung von Prepolymeren oder Polyurethanen.

## Claims

1. Process for the preparation of organic isocyanates containing carbodiimide and/or uretonimine groups by partial carbodiimidization of isocyanate groups with catalysts of the phospholine type and subsequent termination of the carbodiimidization reaction by addition of a silylated acid of the formula X-[Si(CH₃)₃]ₙ, wherein, in the formula
X represents the neutral acid radical such as is obtained from an n-basic acid having a pKa value of not more than 3 by removal of the acid hydrogen atoms, hydrogen halide acids being excluded, and
n denotes an integer from 1 - 3,
**characterized in that** in addition to the silylated acid, a non-silylated acid and/or an acid chloride and/or a sulfonic acid ester is added, wherein optionally halogenated, aliphatic and/or cycloaliphatic and/or aromatic mono-, di- and/or polycarboxylic acids, sulfonic acids, HCl and/or phosphoric acid or mono- and/or diesters thereof are employed as a non-silylated acid and the acid chlorides derived from the optionally halogenated, aliphatic and/or cycloaliphatic and/or aromatic mono-, di- and/or polycarboxylic acids or sulfonic acids and carbamic acids chlorides are employed as an acid chloride.

2. Process according to claim 1, **characterized in that** the addition of a non-silylated acid and/or an acid chloride and/or a sulfonic acid ester is carried out either at the same time as the addition of the silylated acid or in a subsequent step.

3. Process according to one of claims 1 or 2, **characterized in that** the non-silylated acid and/or the acid chloride and/or the sulfonic acid ester are added in amounts of in total between 10 and 1,000 ppm, preferably between 10 and 500, particularly preferably between 50 and 250 ppm, based on the organic isocyanates containing carbodiimide and/or uretonimine groups.

4. Process according to claim 1, **characterized in that** acetic acid, adipic acid, cyclohexanedicarboxylic acid, α-chloropropionic acid, benzoic acid, phthalic acid, isophthalic acid and/or dibutyl phosphate are added as a non-silylated acid.

5. Process according to claim 1, **characterized in that** n-butylcarbamic acid chloride is added as an acid chloride.

6. Process according to one of claims 1 to 3, **characterized in that** p-toluenesulfonic acid methyl ester and/or p-toluenesulfonic acid ethyl ester are employed as a sulfonic acid ester.

7. Organic isocyanates containing carbodiimide and/or uretonimine groups obtainable by the process according to one of claims 1 to 6.

8. Use of the organic isocyanates containing carbodiimide and/or uretonimine groups according to claim 7 for the preparation of isocyanate blends.

9. Use of the organic isocyanates containing carbodiimide and/or uretonimine groups according to claim 7 for the preparation of prepolymers or polyurethanes.

## Revendications

1. Procédé de préparation d'isocyanates organiques présentant des radicaux carbodiimido et/ou urétonimino, par carbodiimidation partielle des radicaux isocyanate avec des catalyseurs de type phospholine, et ensuite arrêt de la réaction de carbodiimidation par l'addition d'un acide silylé de la formule X-[Si(CH₃)₃]ₙ, où dans la formule
X représente un reste acide neutre, comme il est obtenu par élimination des atomes d'hydrogène acides d'un acides n-valent avec un pKa de maximum 3, où les acides halogénohydriques sont exclus, et
n représente un nombre entier allant de 1 à 3,
**caractérisé en ce qu'**en plus de l'acide silylé, un acide non silylé et/ou un chlorure d'acide et/ou un ester d'acide sulfonique est ajouté, où comme acide non silylé, on met en oeuvre un mono-, di- et/ou polyacide carboxylique aliphatique et/ou cycloaliphatique et/ou aromatique le cas échéant halogéné, un acide sulfonique, HCl et/ou un acide phosphorique et respectivement, leur mono- et/ou diester, et comme chlorure d'acide, on met en oeuvre les chlorures d'acide dérivés des mono-, di- et/ou polyacides carboxyliques aliphatiques et/ou cycloaliphatiques et/ou aromatiques le cas échéant halogénés et respectivement, des acides sulfoniques, ainsi que des chlorures d'acide carbamique.

2. Procédé selon la revendication 1, **caractérisé en ce que** l'addition d'un acide non silylé et/ou d'un chlorure d'acide et/ou d'un ester d'acide sulfonique est réalisée simultanément à l'addition de l'acide silylé ou dans une étape suivante.

3. Procédé selon l'une des revendications 1 ou 2, **caractérisé en ce que** l'acide non silylé et/ou le chlorure d'acide et/ou l'ester d'acide sulfonique sont ajoutés en des quantités allant globalement, de 10 à 1000 ppm, de préférence de 10 à 500 et de manière particulièrement préférée, de 50 à 250 ppm, sur base de l'isocyanate organique présentant des radicaux carbodiimido et/ou urétonimino.

4. Procédé selon la revendication 1, **caractérisé en ce que** l'on ajoute comme acide non silylé, l'acide acétique, l'acide adipique, l'acide cyclohexanedicarboxylique, l'acide α-chloropropionique, l'acide benzoïque, l'acide phtalique, l'acide isophtalique et/ou le phosphate de dibutyle.

5. Procédé selon la revendication 1, **caractérisé en ce que** l'on ajoute comme chlorure d'acide, le chlorure de l'acide n-butylcarbamique.

6. Procédé selon l'une des revendications 1 à 3, **caractérisé en ce que** l'on met en oeuvre comme ester d'acide sulfonique, l'ester méthylique de l'acide p-toluènesulfonique et/ou l'ester éthylique de l'acide p-toluènesulfonique.

7. Isocyanate organique présentant des radicaux carbodiimido et/ou urétonimino, obtenu par le procédé selon l'une des revendications 1 à 6.

8. Utilisation de l'isocyanate organique présentant des radicaux carbodiimido et/ou urétonimino selon la revendication 7, pour la préparation de mélanges d'isocyanates.

9. Utilisation de l'isocyanate organique présentant des radicaux carbodiimido et/ou urétonimino selon la revendication 7, pour la préparation de prépolymères ou de polyuréthannes.
